# EUROPEAN PATENT APPLICATION

(11) **EP 2 505 201 A1**
(43) Date of publication of application: **03.10.2012**
(21) Application number: 11160171.2
(22) Date of filing: 29.03.2011
(51) Int. Cl.: A61K 31/49, A23L 1/00, A23L 1/29, A61P 3/04

(54) **Trmp5 inhibitors support body weight reduction without reducing food intake**

(71) Applicant: Nestec S.A., 1800 Vevey (CH)
(72) Inventor: Damak, Sami, 1066, Epalinges (CH); Cettour-Rose, Philippe, 74200, Thonon les Bains (FR); Le Coutre, Johannes, 1009, PULLY (CH)
(74) Representative: Marquardt, Ulf

(57) **Abstract**

The present invention relates to the field of weight management and the prevention and/or treatment of metabolic disorders. In particular, the present invention provides a composition comprising at least one Trpm5 inhibitor which is effective in the treatment of prevention of metabolic disorders or risk factors thereof, such as overweightness or obesity, without having to reduce food intake below metabolic needs.

## Description

The present invention relates to the field of weight management and/or the prevention and/or treatment of metabolic disorders. In particular, the present invention provides a composition comprising at least one Trpm5 inhibitor which is effective in the treatment of prevention of metabolic disorders or risk factors thereof, such as overweightness or obesity, without having to reduce food intake below metabolic needs.

Obesity and its related health conditions constitute a major challenge for today's health care systems. The World Health Organization estimates that approximately 1.6 billion adults are overweight and at least 400 million adults are obese (WHO, 2006, fact sheet N°311). Weight gain and the expansion of white adipose tissue are directly related to excess nutrition and an energy imbalance between consumed and expended calories. The resulting obesity is a key risk factor for disorders referred to as metabolic syndrome, which include significant increases in the risk of developing type 2 diabetes and cardiovascular diseases. Obesity is considered to be preventable, however, healthy weight management can be difficult since the tightly regulated balance of calorie uptake, energy consumption and storage is polymorphic between individuals due to strong variation in their (epi)genetic background, metabolism and life style.

Current interventions against obesity usually require a change in lifestyle including exercise and caloric restriction, modification of nutrient absorption, e.g., by administering the lipase inhibitor orlistat, act as appetite suppressants such as the recently withdrawn drug sibutramine (FDA, 2010) or include medical surgery.

Since change in daily life habits is difficult for most people, these interventions are mostly inefficient with respect to weight loss or do not correspond to a non-invasive, healthy weight management.

One way that is frequently discussed in the art is to combat obesity or overweightness by reducing food intake.

For example, EP 1,968,685 A2 discloses a method and apparatus for reducing obesity. A duodenal/small intestinal insert is disclosed that releases bioactive materials into the intestine via an insert to produce a feeling of satiety. This reduces food intake.

US2003017233 (A1) discloses a meal-equivalent food bar divided into segments equivalent to the sequence of courses in a conventional meal, including appetizer with functional appetite stimulants; main-course with major nutrition ingredients; and dessert with functional appetite depressants. Quinine - because of its bitter taste - is used as an appetite depressant to produce a satiety effect.

United States Patent Application 20080306030 discloses a method of inhibiting food intake comprising administering to a subject an effective amount of a TRPM5 inhibitor in order to reduce food intake.

Reducing food intake to below metabolic needs and energy expenditure will result in weight loss. However, the body will eventually adjust to the low caloric intake with protective measures, will not use all energy from fat cells, but will slow down metabolism instead to conserve energy.

After returning to normal eating habits the body needs time to adjust to the new situation. During this time, the metabolism is still slow and all excess energy from food intake will be stored in fat cells.

This is demotivating for those trying to reduce their weight and often may even lead overall to long-term weight increases despite all efforts to reduce weight

Hence, it would be desirable to be able to manage or reduce weight effectively without having reducing food intake in order to prevent Yo-yo effects.

The present inventors have addressed this need.

Consequently, it was the object of the present invention to improve the state of the art and in particular to provide the art with a composition that allows it to manage body weight, or to reduce body weight in cases of overweightness or obesity, while avoiding the Yo-yo effect, for example, to treat or prevent metabolic disorders.

The present inventors were surprised to see that they could achieve this objective by the subject matter of the independent claim. The dependant claims further develop the subject matter of the present invention.

The present inventors provide a composition that can be used to manage and/or reduce weight without having to reduce food intake.

Hence, weight loss can be achieved while maintaining a food intake that satisfies the body's needs and does not result in a caloric restriction that causes the body to react to the apparent crisis situation, e.g., by slowing down metabolism.

Consequently, Yo-yo effects and the risk to provide insufficient supply of nutrients while reducing food intake are avoided.

The present inventors have incorporated Trpm5 inhibitors into the diet of mice and have found that this leads to a decrease in body weight and fat mass independently of food intake.

Consequently, the present invention relates in part to a composition comprising at least one Trpm5 inhibitor for use in the treatment or prevention of metabolic disorders without reducing food intake.

The present invention also relates to the use of at least one Trpm5 inhibitor in the preparation of a composition for the treatment of prevention of metabolic disorders without reducing food intake.

Trpm5 inhibitors are well known to those skilled in the art. For example, the assay disclosed in U.S. patent application Ser. No. 11/592,180, hereby incorporated by reference in its entirety, can be used to identify compounds that are inhibitors of Trpm5. Also, the assay disclosed in U.S. Patent Application Publication No. 20050019830, hereby incorporated by reference in its entirety, can be used to identify compounds that are inhibitors of Trpm5.

Without reducing food intake may mean that the caloric intake corresponds to the daily caloric needs.

Consequently, the caloric intake does not have to be reduced to a level lower than the daily expenditure, so that any required additional energy is generated from reserves stored in fat cells.

The composition of the present invention may be for humans or animals, such as pet animals for example.

The composition of the present invention may also be for use in supporting weight loss and/or weight maintenance in humans and/or animals.

Supporting weight loss helps to reduce the risk for developing metabolic disorders and diabetes type 2, for example.

Any metabolic disorder may be treated or prevented according to the present invention. For example, the metabolic disorders may be selected from the group consisting of diabetes, hypertension, cardiovascular diseases, and combinations thereof.

In subjects with normal weight the composition can be used to support weight maintenance, in particular at occasions of unusual high caloric intake.

For example, the composition may be used successfully around the holiday seasons.

The composition may also be for use in the treatment of overweightness or obesity.

Both, overweightness and obesity are well-known disorders that represent a significant burden in our society today.

"Overweight" is defined for an adult human as having a BMI between 25 and 30.

"Body mass index" or "BMI" means the ratio of weight in kg divided by the height in metres, squared.

"Obesity" is a condition in which the natural energy reserve, stored in the fatty tissue of animals, in particular humans and other mammals, is increased to a point where it is associated with certain health conditions or increased mortality. "Obese" is defined for an adult human as having a BMI greater than 30.

As detailed above, one advantage of the composition of the present invention is that it can be used to avoid the Yo-yo effect.

Yo-yo effects are also known in the art as weight cycling. The dieter is initially successful in the pursuit of weight loss but does not maintain the reduced weight long-term. Instead the dieter regains its weight - and often times more its starting weight. The dieter then seeks to lose the regained weight, and the cycle begins again.

This cycling is not only inconvenient and psychologically stressful for the dieter but also bears health risks. There are links between body weight variability and negative health outcomes, particularly all-cause mortality and mortality from coronary heart disease. Weight cycling may also have negative psychological and behavioral consequences; studies have reported increased risk for psychopathology, life dissatisfaction, and binge eating (see, e.g., Kelly D. Brownell et al., Arch Intern Med. 1994; 154(12):1325-1330).

Oftentimes, dietary regimens lead to weight loss, but this weight loss is derived from loss of water or protein muscle mass. Dehydration, however, is a serious health risk; and the loss of protein muscle mass is counterproductive to weight loss plans, as muscles are a significant factor in burning calories, and less muscles burn less calories.

The inventors have shown that composition of the present invention is particularly well suited to reduce body fat mass, while keeping the lean body mass constant.

The composition may hence be for use in the reduction of fat mass, in particular white fat mass.

The at least one Trpm5 inhibitor may be provided as pure compound or in any kind of composition, as long as the composition can be administered to the subject in need thereof.

For example, the composition may be selected from the group consisting of food compositions, nutritional compositions, nutraceuticals, powdered nutritional products to be reconstituted in water or milk before consumption, food additives, medicaments, drinks, and pet food.

The composition may be in the form of tablets, capsules, pastilles, chewing gum or a liquid for example. They may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. They may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

Further, they may contain an organic or inorganic carrier material suitable for oral or enteral administration as well as vitamins, minerals trace elements and other micronutrients in accordance with the recommendations of Government bodies such as the USRDA.

For example, the composition may contain per daily dose one or more of the following micronutrients in the ranges given:- 300 to 500 mg calcium, 50 to 100 mg magnesium, 150 to 250 mg phosphorus, 5 to 20 mg iron, 1 to 7 mg zinc, 0.1 to 0.3 mg copper, 50 to 200 µg iodine, 5 to 15 µg selenium, 1000 to 3000 µg beta carotene, 10 to 80 mg Vitamin C, 1 to 2 mg Vitamin B1, 0.5 to 1.5 mg Vitamin B6, 0.5 to 2 mg Vitamin B2, 5 to 18 mg niacin, 0.5 to 2.0 µg Vitamin B12, 100 to 800 µg folic acid, 30 to 70 µg biotin, 1 to 5 µg Vitamin D, 3 to 10 µg Vitamin E.

Any Trpm5 inhibitor or any combination of Trmp5 inhibitors may be used for the purpose of the present invention.

Advantageously, the at least one Trmp5 inhibitor is accepted as a food or pet food ingredient.

For example the Trpm5 inhibitor may be quinine ((R)-(6-methoxyquinolin-4-yl)((2S,4S,8R)- 8-vinylquinuclidin-2-yl)methanol.

The at least one Trmp5 inhibitor may be administered in an amount that is considered acceptable for human or animal consumption.

In therapeutic applications, compositions are administered in an amount sufficient to at least partially cure or arrest the symptoms of the disease and its complications. An amount adequate to accomplish this is defined as "a therapeutically effective dose". Amounts effective for this purpose will depend on a number of factors known to those of skill in the art such as the severity of the disease and the weight and general state of the patient. In prophylactic applications, compositions according to the invention are administered to a patient susceptible to or otherwise at risk of a particular disease in an amount that is sufficient to at least partially reduce the risk of developing a disease. Such an amount is defined to be "a prophylactically effective dose". Again, the precise amounts depend on a number of patient specific factors such as the patient's state of health and weight.

The at least one Trmp5 inhibitor may be administered in the prophylactically effective dose or a therapeutically effective dose.

For example, the compositions of the present invention may comprise at least one Trpm5 inhibitor in an amount of at least 0,1 mg/kg of the composition, of at least 0,5 mg/kg of the composition, or of at least 1 mg/kg of the composition.

The composition of the present invention may be to be administered in an amount corresponding to at least 0,003 mg Trpm5 inhibitor /kg body weight per day, at least 0,1 mg quinine /kg body weight per day, or at least 0,5 mg quinine /kg body weight per day.

For example, if the Trpm5 inhibitor is quinine, the composition of the present invention may be to be administered in an amount corresponding to about 3 mg Trpm5 inhibitor /kg body weight per day to 25 mg Trpm5 inhibitor /kg body weight per day.

The compositions of the present invention may be administered at any time during the day.

Advantageously, they are to be administered briefly before, during or after the meals. For example, they may be to be administered in the timeframe of 1 hour before a meal to 30 minutes after a meal.

Ideally, they are co-administered with the meal. The may be administered during a meal or may be an integral part of the meal.

Trmp5 inhibitors may be provided by any means known to those of skill in the art. They may be synthesized chemically or may be provided from natural sources, e.g., in purified form or in the form of an extract.

Those skilled in the art will understand that they can freely combine all features of the present invention described herein, without departing from the scope of the invention as disclosed. In particular, features described for the use of the present invention may be applied to the composition of the present invention and vice versa.

Further advantages and features of the present invention are apparent from the following Examples and Figures.

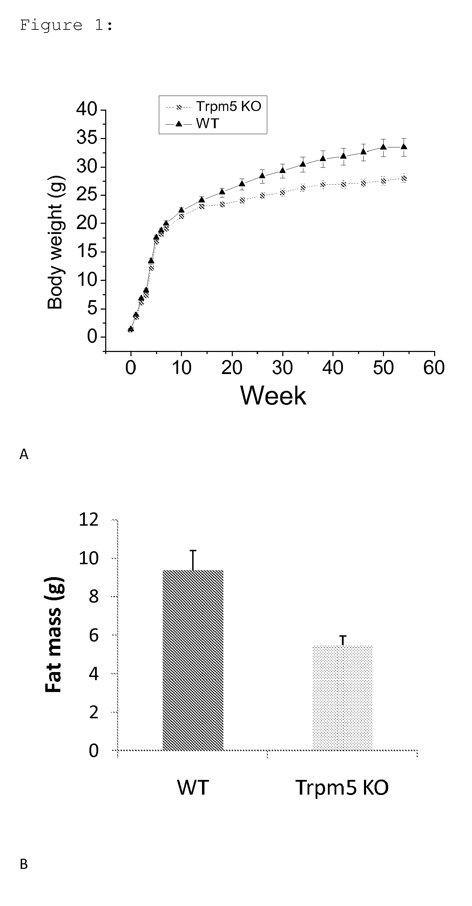

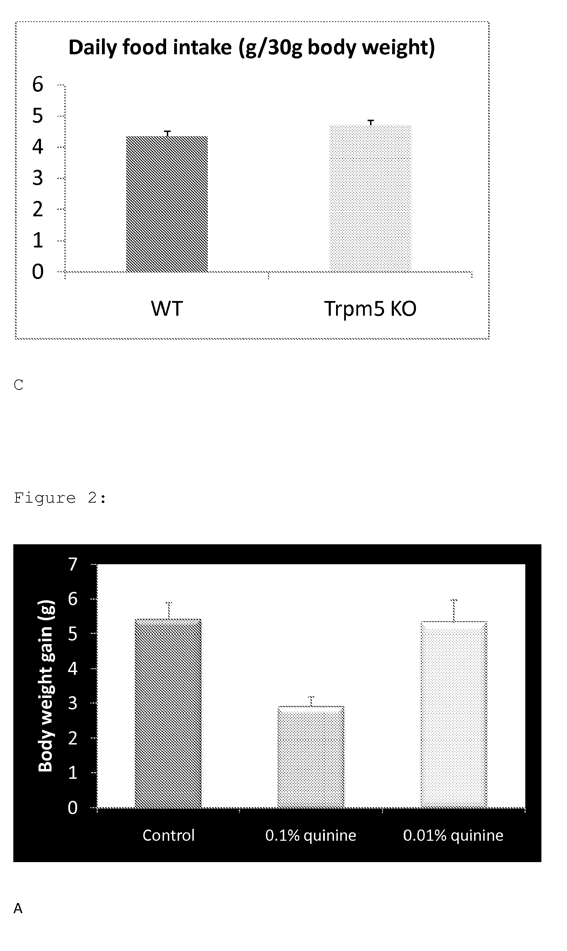

Figure 1 shows the body weight (A), fat mass (B) and average daily food intake (C) during the first year of life of Trpm5 knockout and wild type mice consuming regular diet. Trpm5 knockout mice gained less weight and fat mass than control mice, whereas food intake was not different between the groups.

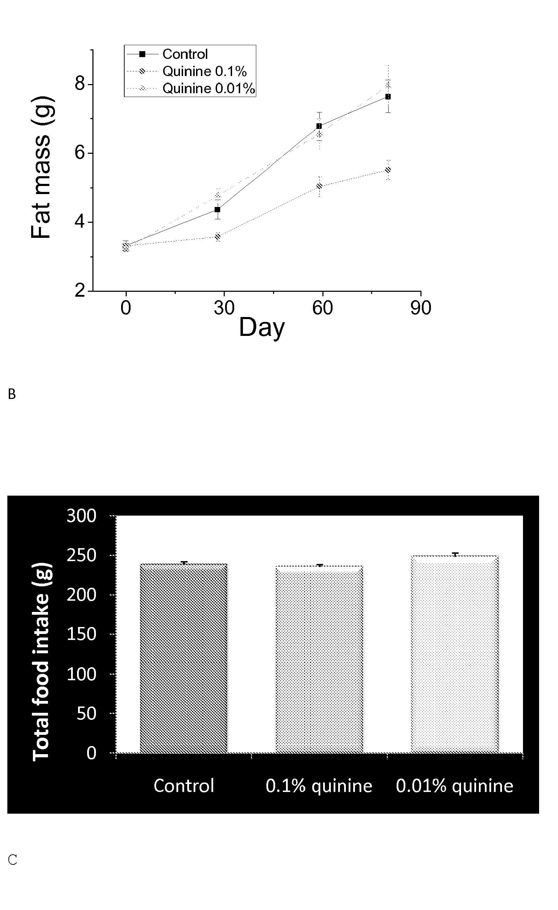

Figure 2 shows the body weight gain (A), fat mass (B) and total food intake (C) over 12 weeks of mice consuming a diet supplemented with 0.1% or 0.01% quinine or a control diet. Mice consuming the 0.1% quinine supplemented diet gained less weight and fat mass than mice consuming control diet, whereas food intake was not different between the groups.

### Example:

### Experiment 1

Mice in which Trpm5 was deleted (Trpm5 knockout mice) were generated by homologous recombination in embryonic stem cells followed by blastocyst injection according to standard methods, as described in Riera et al (2009) J. Neuroscience: 29 p 2654- 2662.

Male and female Trpm5 KO mice (n=42) and wild type littermates(n=27) were weighed weekly from birth for 54 weeks. The mice were fed regular chow (Kliba 3437).

Daily food intake was measured once a week beginning week 28, by weighing the diet at the beginning and end of the week, normalized to the mouse body weight, and averaged over a 6 month period.

Body composition was determined on 53 week-old mice by nuclear magnetic resonance (NMR) using an EchoMRI-900 Body Composition Analyzer (Echo Medical System, LLC, Houston, TX, USA).

### Experiment 2

Three groups of wild type male C57BL6 mice 3 months old , 20 mice per group, were fed either a semi-synthetic (AIN) diet, or AIN diet with 0.1% quinine, or AIN diet with 0.01% quinine. Body weight was measured weekly, body composition was measured monthly, food and fluid intake were measured twice a week over a period of 12 weeks.

### Results

### Experiment 1

Trpm5 KO mice showed a lower body weight than wild type mice (p<0.001, comparing Trpm5 KO and wild type mice across all measurement, Figure 1A).

Nuclear magnetic resonance (NMR) measurement showed that the difference in body weight between Trpm5 KO and WT mice is accounted for by difference in fat mass (Figure 1B). There is no difference between groups in lean mass (Not shown).

There is no significant difference in average daily food intake between groups, which indicates that the difference in body weight is not the result of the Trpm5 KO group eating less diet.

### Experiment 2

Quinine is an inhibitor of Trpm5 (Talavera et al, 2008, FASEB J.:22, 1343-1355). Therefore we investigated whether giving quinine in the diet of wild type mice would mimic the effect of knocking out Trpm5 and lead to a decrease in body weight and fat mass gain in wild type mice.

Body weight gain was significantly lower in the group that consumed the diet with 0.1% quinine (p<0.01), whereas the group which consumed 0.01% quinine was not different from control (Figure 2A).

Nuclear magnetic resonance (NMR) measurement showed that the difference in body weight between the 0.1% quinine and the control groups is accounted for by difference in fat mass. As shown in Figure 2B mean fat mass at the end of the trial was 7.65g ± 0.47 for control, 5.524g ± 0.28 for 0.1% quinine (p<0.01 compared to control) and 7.9675g ± 0.58 for 0.01% quinine. There is no difference between groups in lean mass (Not shown)

Total food intake is shown in Figure 2C. There is no difference in food intake between groups, which indicates that the difference in body weight is not the result of the 0.1% quinine group eating less diet.

To summarize, Trpm5 knockout mice, and mice consuming quinine, an inhibitor of Trpm5, gain less weight and fat mass than wild type mice on a regular diet while consuming an equal amount of diet.

## Claims

1. Composition comprising at least one Trpm5 inhibitor for use in the treatment of prevention of metabolic disorders without reducing food intake.

2. Composition for use according to claim 1 for use in supporting weight loss and/or weight maintenance in humans and/or animals.

3. Composition for use according to one of the preceding claims for use in the treatment of overweightness or obesity.

4. Composition for use according to one of the preceding claims wherein the Yo-yo effect is avoided.

5. Composition for use according to one of the preceding claims for use in the reduction of fat mass.

6. Composition for use according to claim 1, wherein the metabolic disorders are selected from the group consisting of diabetes, hypertension and cardiovascular diseases.

7. Composition for use according to one of the preceding claims wherein the composition is selected from the group consisting of food compositions, nutritional compositions, nutraceuticals, powdered nutritional products to be reconstituted in water or milk before consumption, food additives, medicaments, drinks, and pet food.

8. Composition for use according to one of the preceding claims, wherein the Trpm5 inhibitor is quinine ((R)-(6-methoxyquinolin-4-yl)((2S,4S,8R)- 8-vinylquinuclidin-2-yl)methanol.

9. Composition for use according to one of the preceding claims, wherein the Trpm5 inhibitor is quinine hydrochloride

10. Composition for use according to one of the preceding claims, wherein the Trpm5 inhibitor is quinine sulphate.

11. Composition for use according to one of the preceding claims comprising Trpm5 inhibitor in an amount of at least 0,1 mg/kg of the composition, of at least 0,5 mg/kg of the composition, or of at least 1 mg/kg of the composition.

12. Composition for use according to one of the preceding claims to be administered in an amount corresponding to at least 0,003 mg Trpm5 inhibitor /kg body weight per day, at least 0,1 mg quinine /kg body weight per day, or at least 0,5 mg quinine /kg body weight per day.

13. Composition for use according to one of the preceding claims to be administered in the timeframe of 1 hour before a meal to 30 minutes after a meal.

14. Composition for use according to one of the preceding claims to be administered during a meal.

15. Composition for use according to one of claims 9-12 wherein the quinine is provided from natural sources.
